# EUROPEAN PATENT SPECIFICATION

(11) **EP 3 746 789 B1**
(45) Date of publication and mention of the grant of the patent: **20.04.2022**
(21) Application number: 19701524.1
(22) Date of filing: 25.01.2019
(51) Int. Cl.: G01N 33/574, G01N 33/68

(54) **SCREENING METHOD FOR THE IDENTIFICATION OF CANCER THERAPEUTICS**
SCREENING-VERFAHREN ZUR IDENTIFIZIERUNG VON KREBSTHERAPEUTIKA
MÉTHODE DE CRIBLAGE PERMETTANT L'IDENTIFICATION D'AGENTS THÉRAPEUTIQUES CONTRE LE CANCER

(30) Priority: 29.01.2018 EP 18153933
(43) Date of publication of application: 09.12.2020
(73) Proprietor: Deutsches Krebsforschungszentrum - Stiftung des öffentlichen Rechts / Universität Heidelberg, 69120 Heidelberg (DE)
(72) Inventor: ROSENSTIEL-GOIDTS, Violaine, 76676 Graben-Neudorf (DE); BETHKE, Frederic, 74858 Aglasterhausen (DE); BALSS, Jörg, 69221 Dossenheim (DE); PHILLIPS, Emma, 69214 Eppelheim (DE)
(74) Representative: Krauss, Jan
(86) International application number: PCT/EP2019/051889
(87) International publication number: WO 2019/145496

(56) References cited:
- WO-A2-2008/073382
- US-B2- 8 283 332
- EMMA L. PHILLIPS ET AL: "Abstract 1906: PFKFB4 , much more than just a glycolytic gene", CANCER RESEACRH, vol. 78, no. 13 Supplement, 1 July 2018 (2018-07-01), pages 1906-1906, XP055568982, Proceedings: AACR Annual Meeting 2014; April 5-9, 2014; San Diego, CA ISSN: 0008-5472, DOI: 10.1158/1538-7445.AM2018-1906

## Description

The present invention pertains to a method for identifying anti-cancer compounds. The invention is based on the finding that a direct protein-protein interaction between 6-phosphofructo-2-kinase/fructose-2,6-biphosphatase 4 (PFKFB₄) and (F-box protein 28) FBXO28 silences a ubiquitin E3 ligase activity of FBX028 towards HIFia. Interfering with this protein-protein interaction leads to a strong induction of HIFia proteasomal degradation and cell death in tumors, and therefore, compounds screened according to the present invention harbour therapeutic potential for the treatment of proliferative diseases such as cancer. The invention provides a screening method for cancer therapeutics based on the interaction of PFKFB4 and FBXO28, as well medical applications thereof.

### DESCRIPTION

Cancer is a major lethal disease for humans and is caused by physiologically-uncontrolled cell proliferation which affects normal physiological conditions of human body resulting in serious pathological reactions often leading to death. Although tremendous efforts on cancer studies and treatments have been made, presently, cancer is still the major cause of death to humans. There are multiple approaches to treat cancer patients including surgery, radiation therapy and chemotherapy.

Neoplastic cells preferentially utilize glycolysis to satisfy their increased needs for energy and biosynthetic precursors. The PFKFB enzymes (PFKFB1-4) synthesize fructose-2,6-bisphos-phate (F2,6BP). F2,6BP activates 6-phosphofructo-l -kinase (PFK-1), an essential control point in the glycolytic pathway. Until recently, the PFKFB3 isozyme has been considered the principal source of the increased F2,6BP observed in cancer cells. However, new evidence indicates the co-expression of several PFKFB isozymes in transformed and untransformed tissues, as well as increased expression of the PFKFB4 isoform in several neoplastic cell lines and in tumors.

WO 2008/073382A2 discloses in *vivo* and *in vitro* methods for identifying agents and compounds which inhibit binding of HSP90 and IP6K2, wherein any phenomenon associated with the binding or inhibition, including cell death, subcellular localization, catalytic activity of IP6K2, and IP7 formation can be monitored.

US8283332B2 discloses methods of reducing expression of PFKFB4 and treating cancer in a cell, wherein the methods including contacting a cell with an effective amount of a PFKFB4 inhibitor comprising short hairpin RNA (shRNA) and small interfering RNA (siRNA) inhibitors, and their methods of use.

Thus, there is a continuing need in the art to identify agents with therapeutic potential for the treatment of proliferative disorders. The identification of compounds is urgently needed in order to allow the further development of therapies from the laboratory into the clinical practise. The more agents are known in the art to potentially be useful in therapy, the higher is the probability of novel successful treatment options in the future.

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

This problem is solved in a first aspect by a method for the identification of a compound which is useful as a medicament for the treatment of cancer, the method comprising the steps of:
(a) Providing a candidate compound,
(b) Providing a protein complex comprising PFKFB4 and FBXO28, or fragments or derivatives thereof, wherein PFKFB4 and FBXO28, or the fragments or de-riv-atives thereof, are in direct protein-to-protein interaction with each other (such as binding each other),
(c) Contacting said candidate compound with the protein complex comprising PFKFB4 and FBXO28, or fragments or derivatives thereof, and
(d) Determining whether contacting in (c) results in a change of protein-protein interaction between PFKFB4 and FBXO28, or the fragments or derivatives thereof, optionally by comparison to a control,
wherein in the event of a reduction of protein-protein interaction between PFKFB4 and FBXO28, or the fragments or derivatives thereof, as determined in step (d), the candidate compound is useful as a medicament for the treatment of cancer.

In the context of the present invention the term "PFKFB4" refers to 6-phosphofructo-2-kinase/fructose-2,6-biphosphatase 4 enzyme. The term shall encompass the human version of this protein, but also its homologs, in particular in mouse or rat. Information on PFKFB4 can be derived from the human gene names webpage (https://www.genenames.org/) which the accession number HGNC:8875. The PFKFB4 protein is accessible on the UniProt webpage under the accession number Q16877, and is further, at least the human version, shown in SEQ ID NO: 1 (isoform 1). All other isoforms of the protein shall also be encompassed by the present invention.

In the context of the present invention the term "FBXO28" refers to F-box protein 28. The term shall encompass the human version of this protein, but also its homologs, in particular in mouse or rat. Information on FBX028 can be derived from the human gene names webpage (https://www.genenames.org/) which the accession number HGNC:29046. The FBXO28 protein is accessible on the UniProt webpage under the accession number Q9NVF7, and is further, at least the human version, shown in SEQ ID NO: 2 (isoform 1). All other isoforms of the protein shall also be encompassed by the present invention.

Hence, in some embodiments the derivative or fragment of PFKFB4 is characterized by its ability to be in protein-protein interaction with a full length FBX028 protein.

Hence, in some embodiments the derivative or fragment of FBX028 is characterized by its ability to be in protein-protein interaction with a full length PFKFB4 protein.

In this context, the ability to be in protein-protein interaction is preferably the ability of such a protein derivative or fragment to form an interaction that mimics the native protein-protein interaction between PFKFB4 and FBXO28. Such a native interaction is described herein in the example section. In some preferred embodiments the protein-protein interaction of PFKFB4 and FBX028 is mediated and/or located in the phosphatase domain of PFKFB4, and/or a domain that is in spatial proximity to the phosphatase domain, wherein spatial proximity is within not more than 50 (preferably 40, 30, 20 or 10) amino acid residues N or C terminally apart from at least one amino acid residues located within the PFKFB4 phosphatase domain. Hence, preferably, any derivative or fragment of PFKFB4 preferably in embodiments retains the phosphatase domain mediating the interaction, and/or a domain that is in spatial proximity to the phosphatase domain, wherein spatial proximity is within not more than 50 (preferably 40, 30, 20 or 10) amino acid residues N or C terminally apart from at least one amino acid residues located within the PFKFB4 phosphatase domain, and wherein such domain is mediating the interaction. Most preferably in all aspects and embodiments, the derivative or fragment of PFKFB4 therefore is a PFKFB4 lacking the any domain or sequence but the phosphatase domain, or more exemplary lacking the kinase domain of PFKFB4.

In further preferred embodiments, the screening assay of the invention is a NanoBiT^{®} Assay as described in detail in Dixon et al., (2016) "NanoLuc Complementation Reporter Optimized for Accurate Measurement of Protein Interactions in Cells" (ACS Chem. Biol., 11, 400-408;). However, also other assays known to the skilled artisan and suitable for determining protein-protein interactions may be subject of the present invention.

All references to the databases in context of the invention refer to their respective versions of January 26, 2018.

In context of the invention also any variants or fragments of PFKFB4 and FBX028 shall be encompassed if they have an amino acid with a sequence identity of at least 50, 60, 70, 80, 85, 86, 87, 88, 89, 90,91, 92, 93, 94, 95, 96, 97, 98, 99 or 100% compared to the amino acid sequence human PFKFB4 and FBXO28, respectively. Therefore to SEQ ID NO: 1 or respectively 2 as disclosed herein. Fragments or PFKFB4 and FBXO28 or their variants, shall preferably have a length of at least 30, 40, 50, 80, 100, 150, 200, 300 or more amino acids.

As used herein, the terms "identical" or percent "identity", when used anywhere herein in the context of two or more nucleic acid or protein/polypeptide sequences, refer to two or more sequences or subsequences that are the same or have (or have at least) a specified percentage of amino acid residues or nucleotides that are the same (i.e., at, or at least, about 60% identity, preferably at, or at least, 65%, 70%, 75%, 80%, 85%, 90%, 91%, 92%, 93% or 94%, identity, and more preferably at, or at least, about 95%, 96%, 97%, 98%, 99%, or higher identity over a specified region - preferably over their full length sequences - , when compared and aligned for maximum correspondence over the comparison window or designated region) as measured using a sequence comparison algorithms, or by manual alignment and visual inspection (see, e.g., NCBI web site). In particular for amino acid identity, those using BLASTP 2.2.28+ with the following parameters: Matrix: BLOSUM62; Gap Penalties: Existence: 11, Extension: 1; Neighboring words threshold: 11; Window for multiple hits: 40.

Fragments or derivatives or variants of PFKFB4 and/or FBX028 can be easily tested for the ability to have a protein-protein interaction. Hence, the present invention also disclosed a method for determining whether a protein derivative, variant and/or fragment of PFKFB4 maintains the ability to interact (bind) directly to FBX028 (preferably in full length version). The method comprises a step of contacting the candidate derivative, variant and/or fragment of PFKFB4 with (for example full length) FBXO28 protein, and detecting their interaction, by for example co-immuno precipitation. Also comprised is a method for determining whether a protein derivative, variant and/or fragment of FBXO28 maintains the ability to interact (bind) directly to PFKFB4 (preferably in full length version). The method comprises a step of contacting the candidate derivative, variant and/or fragment of FBXO28 with (for example full length) PFKFB4 protein, and detecting their interaction, by for example co-immuno precipitation.

The above method for testing protein variants/derivatives and/or fragments may in some embodiments form part of the afore described method for the identification of a compound which is useful as a medicament for the treatment of cancer, preferably if the method encompasses the use of any protein derivative, variant and/or fragment of PFKFB4 and/or FBXO28.

The method of the invention may in some embodiments comprising providing the (protein) complex within a biological assay cell, or is provided in a cell-free system. If the screening is performed in contact of a biological assay cell, then human cells such as HEK cells are preferably used.

In general there are no limits to then nature of the candidate compound usable in the screening of the invention. In preferred embodiments the candidate compound is selected from a small molecular compound ("small molecule"), a polypeptide, peptide, glyco-protein, a peptidomimetic, an antigen binding construct (for example, an antibody, an-tibody-like molecule or other antigen binding derivative, or an antigen binding frag-ment thereof), a nucleic acid such as a DNA or RNA, for example an antisense or in-hibitory DNA or RNA, a ribozyme, an RNA or DNA aptamer, RNAi, siRNA, shRNA and the like, including variants or derivatives thereof such as a peptide nucleic acid (PNA), a genetic construct for targeted gene editing, such as a CRISPR/Cas9 construct and/or a guide nucleic acid (gRNA or gDNA) and/or tracrRNA.

The term "small molecule", as used herein, refers to organic or inorganic molecules either synthesized or found in nature, generally having a molecular weight equal to or less than 1000 D, however the definition of small molecule is in some embodiments not limited by this number.

In the art various methods are known to assay protein-protein interactions (binding) qualitatively or quantitatively. The present invention shall encompass all such prior art known methods applicable by the person of ordinary skill. However, in some embodiments, the following approaches may be preferred:
(i) co-immuno precipitation of the interacting proteins,
(ii) a Förster resonance energy transfer (FRET),
(iii) yeast two hybrid assay,
(iv) protein-protein covalent cross-linking,
(v) mass spectroscopy,
(vi) affinity chromatography,
(vii) affinity blotting,
(viii) two-hybrid reconstruction
(ix) reporter gene assays (NanoBiT^{™}),
(x) detection of HIF1a ubiquitylation,
(xi) detection of HIFia degradation,
(xii) immunofluorescent based assays,
(xiii) detection of assay cell viability.

As used herein, the term "Protein-protein interaction" or any grammatically variants of this expression, refers to the close and stable association between proteins. It usually involves the formation of non-covalent chemical bonds such as hydrogen bonds. Direct interaction means that two proteins involved have close contact and form chemical bonds between them. Indirect interaction between two proteins occurs when they do not interact directly with each other but are bound together through interacting with other proteins which in turn interact directly or indirectly with each other. A preferred protein-protein interaction of the invention is mediated and/or located in the PFKFB4 phosphatase domain, or a domain that is in spatial proximity to the phosphatase domain, wherein spatial proximity is within not more than 50 (preferably 40, 30, 20 or 10) amino acid residues N or C terminally apart from at least one amino acid residues located within the PFKFB4 phosphatase domain.

The screening method of the invention is preferably performed in a non-human animal system, *ex-vivo*, or *in-vitro.* With respect to *ex-vivo* uses, the method is preferably done is cell free systems or alternatively in cell culture. In context of the latter, mammalian cells are preferable and in particular human cell lines may be used such as Human Embryonic Kidney cells (HEK).

The idea of the invention in general is the use of the method in the production and identification of anti-cancer therapeutics. Cancer in context of the invention is therefore preferably a PFKFB4-expressing cancer, preferably a cancer associated with an elevated expression of PFKFB4.

Preferably the cancer is any tumor or cancer disease, preferably selected from a liquid or solid tumor, and preferably is lung cancer, bladder cancer, ovarian cancer, uterine cancer, endome-trial cancer, breast cancer, liver cancer, pancreatic cancer, stomach cancer, cervical cancer, lymphoma, leukemia, acute myeloid leukemia, acute lymphocytic leukemia, salivary gland cancer, bone cancer, brain cancer, colon cancer, rectal cancer, colorectal cancer, kidney cancer, skin cancer, melanoma, squamous cell carcinoma, pleomorphic adenoma, hepatocellular carcinoma, and/or adenocarcinoma. Preferred cancers are glioblastoma, breast, prostate or lung cancer.

Yet a further aspect of the invention then pertains to a compound for use in a method of treating a cancer in a subject, the method comprising the step of interrupting in cell associated with the cancer in the subject the protein-protein interaction between PFKFB4 and FBXO28.

The cancer is preferably a cancer as defined herein above.

A subject according to the invention is preferably a mammal, preferably a human patient suffering from cancer and in need of a treatment.

The treatment method of the invention preferably comprises the administration to the subject of a therapeutically effective amount of the compound which specifically reduces the protein-protein interaction between PFKFB4 and FBX028 in a cell associated with the cancer. The compound is preferably a compound as identified according to the herein disclosed invention.

The present invention will now be further described in the following examples with reference to the accompanying figures and sequences, nevertheless, without being limited thereto. In the Figures:
- **Figure 1:**: TMA and Western blots
- **Figure 2:**: (A) Luminescent image of animals stably ex-pressing the luciferase gene and treated with doxycycline for 6 weeks. (B) Kaplan Meier curves of the animals treated with doxycycline expressing shNT (black) or shPFKFB4 (grey) and non-treated animals (dashed). (C) Tumor size as determined by luminescent imaging, expressed in total flux (photons/second). (D) Scanned H and E stained sections of mouse brains from the non-treated and the shNT expressing groups using the Leica ImageScope. Whole brain, 10-fold and 40-fold magnification are presented.
- **Figure 3:**: PFKFB4 is involved in HIF transcriptional function
- **Figure 4:**: Knockdown of PFKFB4 impairs HIF stability
- **Figure 5:**: FBX028 interacts with PFKFB4
- **Figure 6:**: FBX028 is required for HIFia stability
- **Figure 7:**: shows a NanoBiT^{®} assay for screening candidate compounds.

And in the sequences:
**SEQ ID NO: 1**
**SEQ ID NO: 2**

### EXAMPLES

### Example 1: Tumour-specific expression of PFKFB4

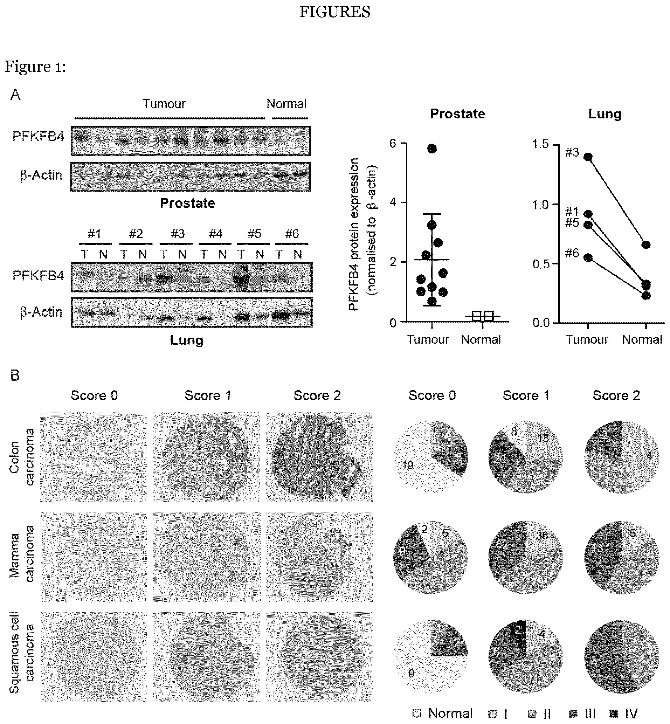

Recent studies have showed the importance of the key glycolysis gene PFKFB4 for the survival of different tumor cells in vitro and in vivo, highlighting its potential as therapeutic target. To investigate the endogenous protein expression level within different cancer entities, the inventors have developed a new PFKFB4 antibody that is suitable for Western blot and immuno-histochemistry, allowing high-throughput staining of Tissue Microarrays (TMAs). As displayed in Figure 1A, prostate tumor protein samples showed a significantly higher level of PFKFB4 expression than normal samples. Similarly, tumor and normal paired samples from lung cancer patients showed a marked difference of PFKFB4 expression level (Figure 1A). As depicted in Figure 1B, the level of PFKFB4 protein expression is ranging from not expressed to high expressed, normal tissues being mostly negative for PFKFB4. Interestingly, the level of expression correlated with the grading of the different tumor, irrespective of its tissue origin, suggesting that PFKFB4 could be involved in the maintenance and growth of tumors.

### Example 2: Knockdown of PFKFB4 impairs GSC viability in vivo

In order to verify the effect of PFKFB4 silencing on the tumor growth in vivo, we used a xeno-graft mouse model. For this purpose, we generated inducible expression constructs encoding an shRNA targeting PFKFB4 and a non-target shRNA as negative control. Stably transduced GSCs (NCH421k_TetONshPFKFB4 and NCH421k_TetONshNT) were treated for three days with doxycycline and the knockdown of PFKFB4 was characterized at the protein level. In order to allow the monitoring of the tumor growth in vivo, the inducible GSC lines were stably transduced with a luciferase-expressing construct.

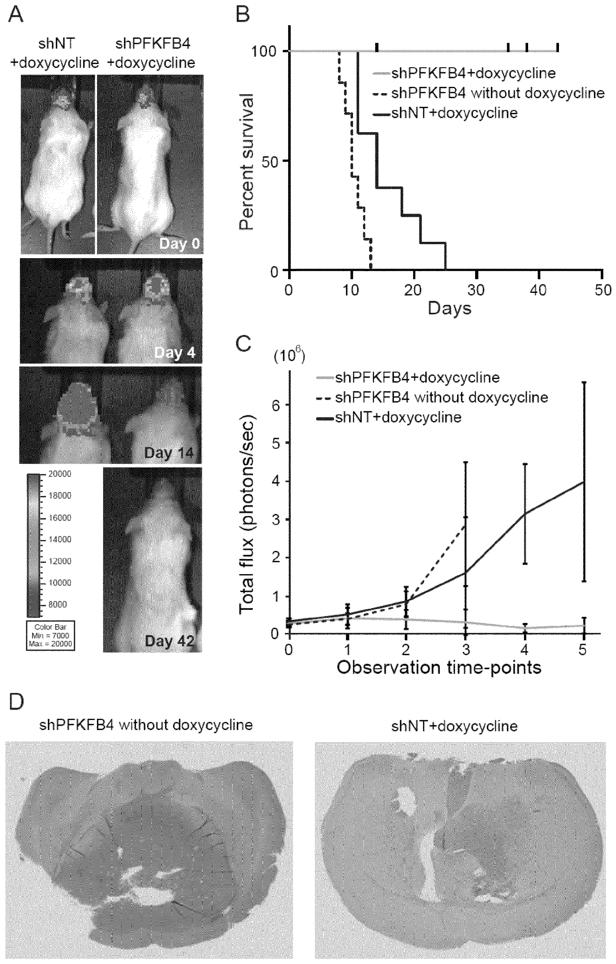

Two groups of 8 and 14 animals were orthotopically transplanted with 100.000 NCH421k_Luc_TetONshNT and NCH421k_Luc_TetONshPFKFB4 cells respectively. The tumor growth expressed as luminescent signal was monitored twice a week. Apparition of the signal of sufficient size (ca. 200.000 flux/photon/second) determined the start of doxycycline treatment. Animals were randomly separated in three groups, namely shNT_dox treated (n=8), shPFKFB4_dox treated (n=7) and shPFKFB4_untreated (n=7). Pictures of the animals were taken twice a week (Figure 2A). Animals expressing shPFKFB4 upon doxycycline treatment showed a significantly better survival than the control animals (non-treated animals and shNT expressing animals) (Figure 2B).

Knockdown of PFKFB4 reduced significantly tumor size overtime as observed by biolumines-cent intensity, ultimately leading to the loss of tumor cells about three weeks after treatment (Figure 2C). Indeed, tumor could be observed in the brain of the sacrified sick animals (non-treated and doxycycline treated shNT) by H and E staining (Figure 2D), while most of the shPFKFB4 doxycycline-treated mice were tumor-free. Residual tumors of shPFKFB4_dox treated animals still expressed PFKFB4, suggesting that the doxycycling induction of shPFKFB4 was not completely efficient in these tumors as confirmed by immunofluorescence.

### Example 3: Impact of PFKFB4 silencing on PDK1 expression

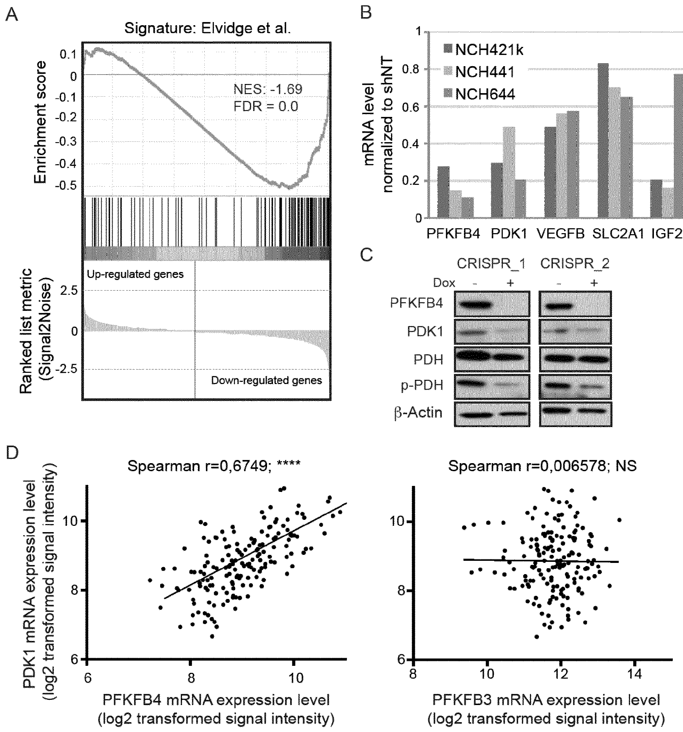

As shown *in vitro* and in *vivo,* the impact of PFKFB4 silencing is particularly high for GSC viability. In order to determine its effect on the regulation of the expression of other genes, the gene expression of three different GSC lines (NCH421k, NCH4**4**1 and NCH644) transduced with pLKO_shPFKFB4 and pLKO_shNT was profiled. Interestingly, knockdown of PFKFB4 led to a decrease of the HIFia gene signature, as determined by Gene Set Enrichment Analysis (GSEA) (Figure 3A). Among the numerous known target of HIFia the inventors identified, such as LDHA, CA9 and IGF2, the Pyruvate Dehydrogenase Kinase 1 (PDK1) showed the strongest reduction upon PFKFB4 knockdown, which was verified by qRT-PCR (Figure 3B). PDKi is a key enzyme that regulates the fate of pyruvate within the glycolytic pathway by phosphorylating and thereby inhibiting the Pyruvate Dehydrogenase (PDH). In order to verify that the knockdown of PFKFB4 also affected the phosphorylation of PDKi's target, the inventors performed western blot analysis upon knockout of PFKFB4 using CRISPR. As depicted in Figure 3C, the level of phosphorylated PDH decreased upon PFKFB4 knockdown.

Because the effect of PFKFB4 silencing on PDKi expression was significant, the endogenous expression of both genes was investigated in a cohort of glioblastoma patients (n=154), available from The Cancer Genome Atlas (TCGA). Notably, PDKi showed the highest expression correlation with PFKFB4 (R=0,67) (Figure 3D), a phenomenon that seemed to be specific to that PFK2/FBP2 isoform. Indeed, none of the other isoforms that are expressed in glioblastoma samples showed any correlation with PDKi expression (Figure 3D).

### Example 4: PFKFB4 is involved in the regulation of HIFia protein levels

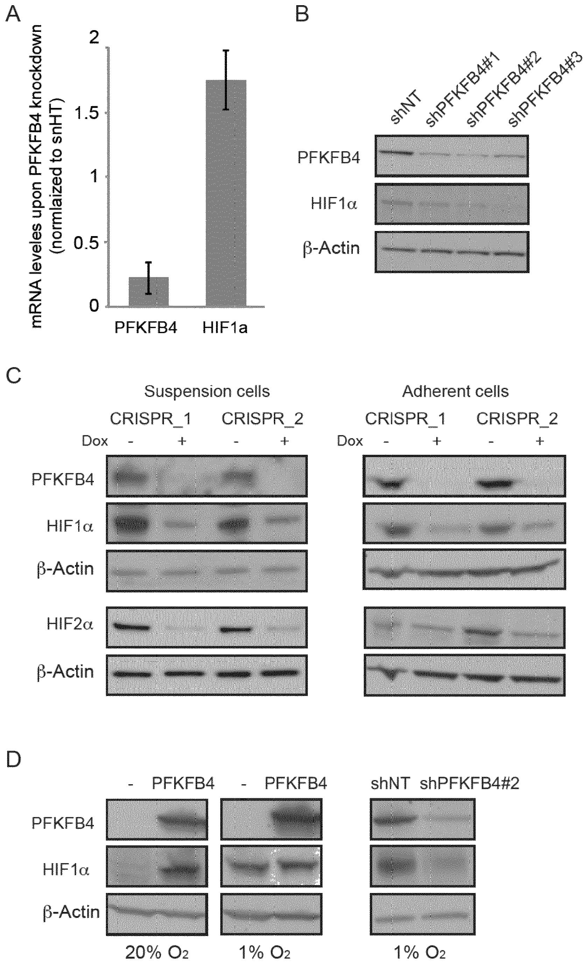

As highlighted by the gene expression profiling of PFKFB4 knockdown GSC lines, PFKFB4 seems to be involved in the regulation of the expression of genes that are targets of the HIFia transcription factor. In order to investigate the potential role of PFKFB4 on the regulation of HIFia expression, qRT-PCR on PFKFB4 knockdown GSCs was performed. As shown in Figure 4A, no decrease of HIFia mRNA could be observed upon PFKFB4 knockdown. Indeed, it seems that upon silencing, HIFia is upregulated at the mRNA level. However, the knockdown of PFKFB4 led to a strong decrease of HIFia at the protein level (Figure 4B), suggesting that PFKFB4 is involved post-transcriptionally in HIFia regulation. This phenomenon was confirmed by knockout of PFKFB4 using two different CRISPR guide RNAs (Figure 4C).

As GSCs are cultivated as neurospheres, which could lead to different level of oxygen and nutrient availability, thereby influencing the dependence on HIFia transcription factor, the inventors performed a PFKFB4 knockout on adherent GSCs. Interestingly, even on these normoxic conditions, HIFia is strongly expressed, suggesting that HIFia protein levels are dependent on PFKFB4 expression, irrespective of the culture conditions (Figure 4C). Furthermore, the overexpression of PFKFB4 in HEK293 cells led to the up-regulation of HIFia, while its knockdown under hypoxic conditions decreased HIFia protein levels (Figure 4D).

### Example 5: Identification of a new E3 ubiquitin ligase of HIFia

As both proteins do not directly interact with each, the inventors performed mass spectrometry of immunoprecipitated PFKFB4 samples to find binding partners of PFKFB4 that could be involved in the mechanisms stabilizing HIFia.

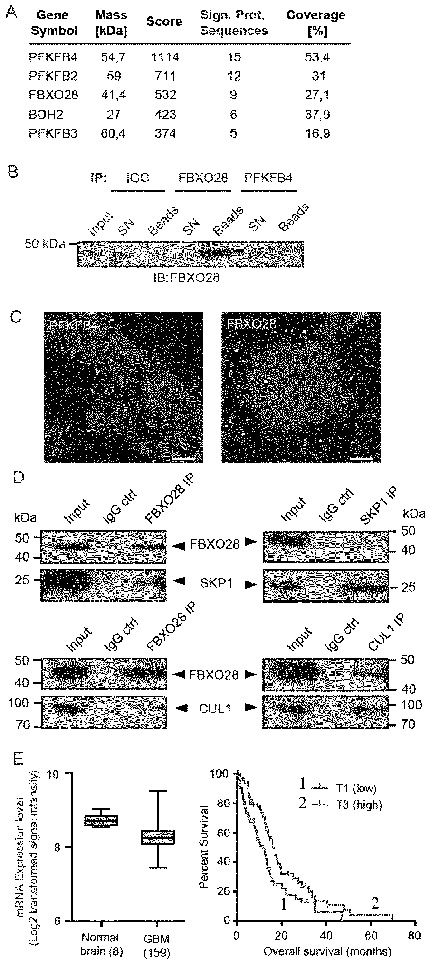

Proteins showing more than two signature sequences are listed in Figure 5A. The binding of FBX028 to PFKFB4 was verified by coIP and by Yeast-Two-Hybrid (Figure 5B). In addition, the cytoplasmic localization of both proteins was verified by immunofluorescence (Figure 5C). FBX028 is a member of the F-Box protein family and is thought to be part of the SCF complex formed by SKPi, cullin and F-box proteins, as shown by coIP (Figure 5D), acting as ubiquitin ligases. Interestingly, unlike PFKFB4, FBXO28 mRNA expression is decreased in glioblastoma as compared to normal brain and patients with a lower expression have a better survival (Figure 5E).

### Example 6: PFKFB4 binds to FBXO28 to inhibit HIFia ubiquitylation and degradation

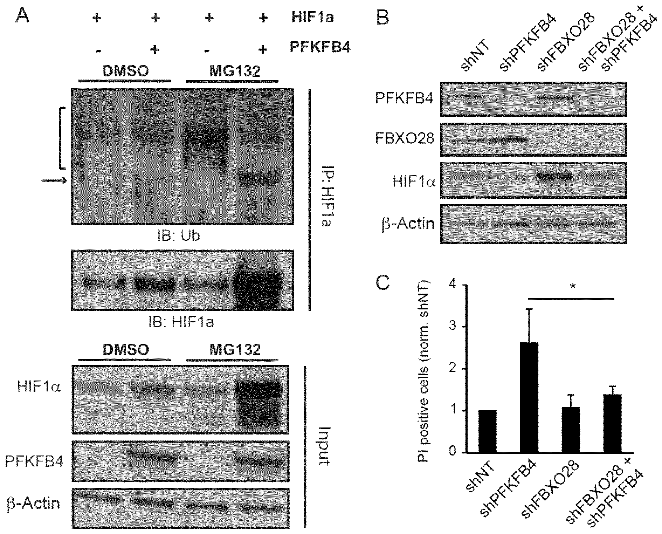

To verify the role of PFKFB4 in the ubiquitylation of HIFia, the inventors performed immunoblotting using ubiquitin antibody on immunoprecipitated HIFia upon MG132 treatment. As highlighted in Figure 6A, the ubiquitylation of HIFia is decreased in cells overexpressing PFKFB4. Next, the importance of the link between FBX028 and for HIFia protein expression and GSCs survival PFKFB4 was identified. In that respect, PFKFB4 was knocked down solely or in combination with FBX028 silencing in GSCs and FACS analysis was performed. The effect on HIF1a protein level was verified by Western blot. As shown in Figure 6B, knockdown of PFKFB4 decreased the protein level of HIFia while silencing of FBX028 alone did not reduce it. However, silencing of both FBX028 and PFKFB4 in the GSCs rescued the level of HIFia. The rescue was also seen at the phenotypic level (Figure 6C).

Taken together, these results emphasize the potential role of PFKFB4 to protect HIFia from the SCF complex, enabling the expression of HIF target genes in glioblastoma stem-like cells.

### Example 7: Development of a screening method to identify small-compound inhibitor to inhibit PFKFB4 function

The NanoBiT^{®} assay from Promega is widely used to investigate protein-protein interactions.

The NanoBiT Assay is described in detail in Dixon et al., (2016) "NanoLuc Complementation Reporter Optimized for Accurate Measurement of Protein Interactions in Cells" (ACS Chem. Biol., 11, 400-408).

The inventors adapted this method to develop a cellular screening assay in order to investigate the interaction of PFKFB4 with FBXO28. In that respect, both genes were cloned into pLVX vectors containing the four NanoBiT^{®} versions (pLVX1.1-N[TK/LgBiT], pLVX2.1-N[TK/SmBiT], pLVX1.1-C[TK/LgBiT] and pLVX2.1-C[TK/SmBiT]), allowing the small and large BiT tags to be at the N- or C-terminal of both proteins of interest. As negative control, a vector containing a HaloTag protein cloned to the small BiT was used in combination with the large BiT cloned to either FBXO28 or PFKFB4.

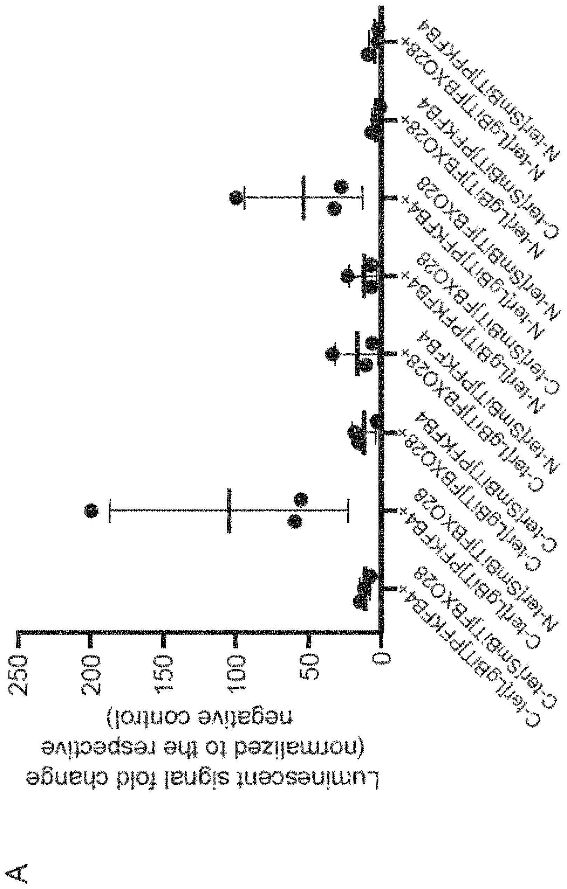

All vectors were transfected in HEK293 cells and the combinations were tested as depicted in Figure 7A. The luminescent signal was detected with the Nano-Glo^{®} Luciferase Assay System from Promega. The interaction was considered positive if the signal was at least 10-fold of the respective negative control. The combination of the vector containing PFKFB4 tagged at the C-terminal with the Large BiT with the vector expressing FBX028 tagged at the N-terminal with the small BiT (see figure 7A, 2^{nd} column) gave the highest signal and was therefore selected for further validation.

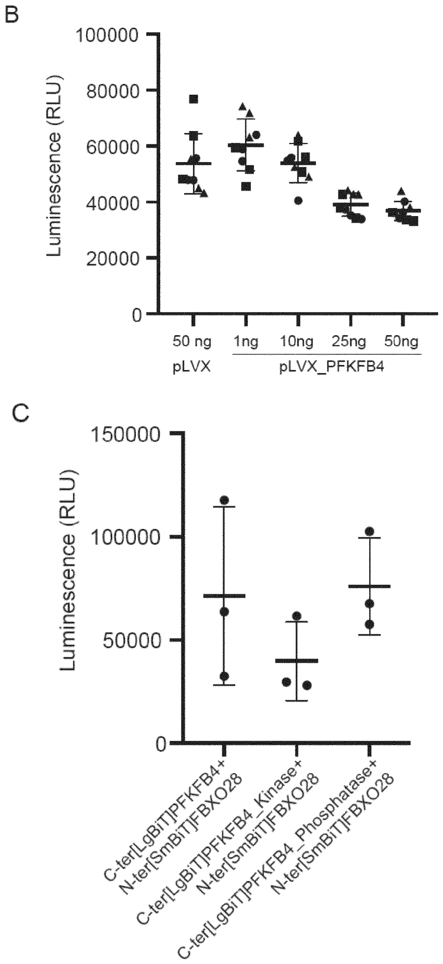

In order to verify the specificity of the assay, the inventors transfected HEK293 cells with the combination showing the best results (C-ter[LgBiT]PFKFB4+N-ter[SmBiT]FBXO28) together with increasing concentration of a vector overexpressing untagged PFKFB4. As shown in Figure 7B, the signal given by the interaction of tagged PFKFB4 and FBX028 decreased upon addition of untagged PFKFB4 which competes with tagged PFKFB4 to form the interacting complex. This is a clear indication that a candidate interacting compound (here represented by the untagged PFKFB4) in a screening assay would yield signals indicative for the impairment of the interaction between both proteins.

Finally, by transfecting different truncated versions of tagged PFKFB4 together with tagged FBXO28, the inventors were able to determine that the interaction is reduced if the phosphatase domain is removed and therefore, that the site of interaction between both proteins is most likely located within the phosphatase domain of PFKFB4 (Figure 7C).

## Claims

1. A method for the identification of a compound which is useful as a medicament for the treatment of cancer, the method comprising the steps of:
(a) Providing a candidate compound,
(b) Providing a protein complex comprising PFKFB4 and FBXO28, or fragments or derivatives thereof, wherein PFKFB4 and FBXO28, or the fragments or derivatives thereof, are in direct protein-to-protein interaction with each other (such as binding each other),
(c) Contacting said candidate compound with the protein complex comprising PFKFB4 and FBXO28, or fragments or derivatives thereof, and
(d) Determining whether contacting in (c) results in a change of protein-protein interaction between PFKFB4 and FBXO28, or the fragments or derivatives thereof, optionally by comparison to a control,
wherein in the event of a reduction of protein-protein interaction between PFKFB4 and FBXO28, or the fragments or derivatives thereof, as determined in step (d), the candidate compound is useful as a medicament for the treatment of cancer.

2. The method according to claim 1, wherein the complex is provided within a biological assay cell, or is provided in a cell-free system.

3. The method according to claim 1 or 2, wherein the candidate compound is selected from a small molecular compound ("small molecule"), a polypeptide, peptide, glycoprotein, a peptidomimetic, an antigen binding construct (for example, an antibody, antibody-like molecule or other antigen binding derivative, or an antigen binding fragment thereof), a nucleic acid such as a DNA or RNA, for example an antisense or inhibitory DNA or RNA, a ribozyme, an RNA or DNA aptamer, RNAi, siRNA, shRNA and the like, including variants or derivatives thereof such as a peptide nucleic acid (PNA), a genetic construct for targeted gene editing, such as a CRISPR/Cas9 construct and/or a guide nucleic acid (gRNA or gDNA) and/or tracrRNA.

4. The method according to any one of claims 1 to 3, wherein the determining in step (d) involves at least one of:
(i) co-immuno precipitation of the interacting proteins,
(ii) a Förster resonance energy transfer (FRET),
(iii) yeast two hybrid assay,
(iv) protein-protein covalent cross-linking,
(v) mass spectroscopy,
(vi) affinity chromatography,
(vii) affinity blotting,
(viii) two-hybrid reconstruction
(ix) reporter gene assays (NanoBiT^{™}),
(x) detection of HIFia ubiquitylation,
(xi) detection of HIFia degradation,
(xii) immunofluorescent based assays,
(xiii) detection of assay cell viability.

5. The method according to any one of claims 1 to 4, which is performed in a non-human animal system, *ex-vivo*, or *in-vitro*, preferably in a human cell line such as Human Embryonic Kidney cells (HEK).

6. The method according to any one of claims 1 to 5, wherein the derivative or fragment of PFKFB4 is **characterized by** its ability to be in protein-protein interaction with a full length FBXO28 protein.

7. The method according to any one of claims 1 to 6, wherein the derivative or fragment of FBXO28 is **characterized by** its ability to be in protein-protein interaction with a full length PFKFB4 protein.

8. The method according to claim 6 or 7, wherein the ability to be in protein-protein interaction is the ability of an interaction that mimics the native protein-protein interaction between PFKFB4 and FBXO28.

9. The method according to any one of claims 1 to 8, wherein the cancer is a PFKFB4-expressing cancer, preferably a cancer associated with an elevated expression of PFKFB4, such as glioblastoma, breast, prostate or lung cancer.

10. A compound for use in a method of treating a cancer in a subject, the method comprising the step of interrupting in cell associated with the cancer in the subject the protein-protein interaction between PFKFB4 and FBXO28.

11. The compound for use according to claim 10, wherein the cancer is a cancer **characterized by** the expression of PFKFB4 and FBXO28.

12. The compound for use according to claim 10 or 11, wherein the subject is a mammal, preferably a human patient suffering from cancer and in need of a treatment.

13. The compound for use according to any one of claims 10 to 12, wherein the method comprising the administration to the subject of a therapeutically effective amount of the compound which specifically reduces the protein-protein interaction between PFKFB4 and FBXO28 in a cell associated with the cancer.

14. The compound for use according to any one of claims 10 to 13, wherein the compound is a compound as identified according to a method of any one of claims 1 to 9.

## Patentansprüche

1. Verfahren zur Identifizierung einer Verbindung, die als ein Medikament zur Behandlung von Krebs brauchbar ist, wobei das Verfahren die Schritte umfasst von:
(a) Zur Verfügung stellen einer Kandidatenverbindung,
(b) Zur Verfügung stellen eines Proteinkomplexes umfassend PFKFB4 und FBXO28 oder Fragmenten oder Derivaten davon, wobei PFKFB4 und FBXO28 oder die Fragmente oder Derivate davon, in direkter Protein-zu-Protein-Interaktion miteinander vorliegen (wie zum Beispiel einander binden),
(c) In Kontakt bringen der Kandidatenverbindung mit dem Proteinkomplex umfassend PFKFB4 und FBXO28 oder den Fragmenten oder Derivaten davon, und
(d) Bestimmen ob das in Kontakt bringen in (c) einer Veränderung der Protein-zu-Protein-Interaktion zwischen PFKFB4 und FBXO28 oder den Fragmenten oder Derivaten davon resultiert, gegebenenfalls durch Vergleich mit einer Kontrolle,
wobei im Falle einer Reduktion der Protein-zu-Protein-Interaktion zwischen PFKFB4 und FBXO28 oder den Fragmenten oder Derivaten davon, wie bestimmt in Schritt (d), die Kandidatenverbindung als ein Medikament zur Behandlung von Krebs brauchbar ist.

2. Verfahren nach Anspruch 1, wobei der Komplex innerhalb einer biologischen Testzelle zur Verfügung gestellt wird, oder in einem zellfreien System zur Verfügung gestellt wird.

3. Verfahren nach Anspruch 1 oder 2, wobei die Kandidatenverbindung ausgewählt ist aus einer Verbindung mit kleinem Molekulargewicht ("kleines Molekül"), einem Polypeptid, Peptid, Glycoprotein, einem Peptidomimetik, einem Antigen-bindenden Konstrukt (wie zum Beispiel einem Antikörper, Antikörper-ähnlichen Molekül oder anderen Antigen-bindenden Derivaten oder einem Antigen-bindenden Fragment davon), einer Nukleinsäure, wie zum Beispiel einer DNA oder RNA, zum Beispiel einer antisense oder inhibitorischen DNA oder RNA, einem Ribozym, einem RNA oder DNA Aptamer, RNAi, siRNA, shRNA und ähnlichem, einschließlich Varianten oder Derivaten davon, wie zum Beispiel einer Peptid-Nukleinsäure (PNA), einem genetischen Konstrukt zum gezielten Gene-Editing, wie zum Beispiel einem CRISPR/Cas9 Konstrukt und/oder a Guide-Nukleinsäure (gRNA oder gDNA) und/oder tracrRNA.

4. Verfahren nach einem der Ansprüche 1 bis 3, wobei das Bestimmen in Schritt (d) mindesten Eines einschließt von:
(i) Co-Immunpräzipitation der interagierenden Proteine,
(ii) einen Förster Resonanz-Energietransfer (FRET),
(iii) Hefe Zwei-Hybrid Assay,
(iv) Protein-Protein kovalente Kreuzvernetzung,
(v) Massenspektroskopie,
(vi) Affinitätschromatographie,
(vii) Affinitätsblotting,
(viii) Zwei-Hybrid-Rekonstruktion
(ix) Reporter Gentests (NanoBiT^{™}),
(x) Nachweis von HIF1a Ubiquitinylierung,
(xi) Nachweis von HIF1a Abbau,
(xii) Immunfluoreszenz-basierte Assays,
(xiii) Nachweis von Lebensfähigkeit der Testzelle.

5. Verfahren nach einem der Ansprüche 1 bis 4, das in einem nicht-menschlichen Tiersystem, *ex-vivo* oder *in-vitro* durchgeführt wird, bevorzugt in einer menschlichen Zelllinie wie zum Beispiel Menschlichen Embryonal-Nierenzellen (HEK).

6. Verfahren nach einem der Ansprüche 1 bis 5, wobei das Fragment oder Derivat von PFKFB4 durch seine Fähigkeit in einer Protein-Protein Interaktion mit einem Volllängen-FBXO28 Protein vorzuliegen gekennzeichnet ist.

7. Verfahren nach einem der Ansprüche 1 bis 6, wobei das Derivat oder Fragment von FBXO28 durch seine Fähigkeit in einer Protein-Protein Interaktion mit einem Volllängen-PFKFB4 Protein vorzuliegen gekennzeichnet ist.

8. Verfahren nach Anspruch 6 oder 7, wobei die Fähigkeit in einer Protein-Protein Interaktion vorzuliegen die Fähigkeit einer Interaktion ist, welche die native Protein-Protein Interaktion zwischen PFKFB4 und FBXO28 nachahmt.

9. Verfahren nach einem der Ansprüche 1 bis 8, wobei der Krebs ein PFKFB4-exprimierender Krebs ist, bevorzugt ein Krebs assoziiert mit einer erhöhten Expression von PFKFB4, wie zum Beispiel Glioblastom, Brust-, Prostata- oder Lungenkrebs.

10. Verbindung zur Verwendung in einem Verfahren zur Behandlung einer Krebserkrankung in einem Subjekt, wobei das Verfahren den Schritt umfasst von Unterbrechen in einer Zelle, die mit dem Krebs in dem Subjekt assoziiert ist, die Protein-Protein Interaktion zwischen PFKFB4 und FBXO28.

11. Verbindung zur Verwendung nach Anspruch 10, wobei der Krebs ein Krebs ist, der durch die Expression von PFKFB4 und FBXO28 gekennzeichnet ist.

12. Verbindung zur Verwendung nach Anspruch 10 oder 11, wobei das Subjekt ein Säugetier ist, bevorzugt ein menschlicher Patient, der an Krebs leidet und einer Behandlung bedarf.

13. Verbindung zur Verwendung nach einem der Ansprüche 10 bis 12, wobei das Verfahren die Verabreichung an das Subjekt einer therapeutisch effektive Menge der Verbindung, die spezifisch die Protein-Protein Interaktion zwischen PFKFB4 und FBXO28 in einer Zelle reduziert die mit dem Krebs assoziiert ist, umfasst.

14. Verbindung zur Verwendung nach einem der Ansprüche 10 bis 13, wobei die Verbindung eine Verbindung wie gemäß eines Verfahren nach einem der Ansprüche 1 bis 9 identifiziert ist.

## Revendications

1. Méthode pour l'identification d'un composé qui est utile en tant que médicament pour le traitement du cancer, la méthode comprenant les étapes de :
(a) Fournir un composé candidat,
(b) Fournir un complexe protéique comprenant PFKFB4 et FBXO28, ou des fragments ou des dérivés de ceux-ci, dans lequel PFKFB4 et FBXO28, ou les fragments ou les dérivés de ceux-ci, sont en interaction directe protéine à protéine l'un avec l'autre (comme une liaison l'un avec l'autre),
(c) Mise en contact dudit composé candidat avec le complexe protéique comprenant PFKFB4 et FBXO28, ou des fragments ou dérivés de ceux-ci, et
(d) Déterminer si la mise en contact en (c) entraîne un changement de l'interaction protéine-protéine entre PFKFB4 et FBXO28, ou les fragments ou dérivés de ceux-ci, éventuellement par comparaison avec un témoin,
dans lequel, dans le cas d'une réduction de l'interaction protéine-protéine entre PFKFB4 et FBXO28, ou les fragments ou dérivés de ceux-ci, comme déterminé dans l'étape (d), le composé candidat est utile comme médicament pour le traitement du cancer.

2. Méthode selon la revendication 1, dans laquelle le complexe est fourni dans une cellule d'essai biologique, ou est fourni dans un système acellulaire.

3. Méthode selon la revendication 1 ou 2, dans laquelle le composé candidat est choisi parmi un petit composé moléculaire ("petite molécule"), un polypeptide, un peptide, une glycoprotéine, un peptidomimétique, une construction de liaison à un antigène (par exemple, un anticorps, une molécule semblable à un anticorps ou un autre dérivé de liaison à un antigène, ou un fragment de liaison à un antigène de celui-ci), un acide nucléique tel qu'un ADN ou un ARN, par exemple un ADN ou un ARN antisens ou inhibiteur, un ribozyme, un aptamère d'ARN ou d'ADN, un ARNi, un pARNi, un shARN et similaires, y compris des variantes ou des dérivés de ceux-ci tels qu'un acide nucléique peptidique (ANP), une construction génétique pour l'édition ciblée de gènes, telle qu'une construction CRISPR/Cas9 et/ou un acide nucléique guide (gARN ou gADN) et/ou un tracrARN.

4. Méthode selon l'une quelconque des revendications 1 à 3, dans laquelle la détermination à l'étape (d) implique au moins l'un des éléments suivants :
(i) co-immunoprécipitation des protéines en interaction,
(ii) un transfert d'énergie par résonance de Förster (TERF),
(iii) test de levure à deux hybrides,
(iv) réticulation covalente protéine-protéine,
(v) spectroscopie de masse,
(vi) chromatographie d'affinité,
(vii) transfert d'affinité,
(viii) reconstruction bi-hybride
(ix) tests de gènes rapporteurs (NanoBiT^{™}),
(x) détection de l'ubiquitylation de HIF1a,
(xi) détection de la dégradation de HIF1a,
(xii) tests basés sur l'immunofluorescence,
(xiii) détection de la viabilité cellulaire de l'essai.

5. Méthode selon l'une quelconque des revendications 1 à 4, qui est mise en œuvre dans un système animal non humain, *ex-vivo*, ou *in-vitro*, de préférence dans une lignée cellulaire humaine telle que les cellules Rénales Embryonnaires Humaines (REH).

6. Méthode selon l'une quelconque des revendications 1 à 5, dans laquelle le dérivé ou fragment de PFKFB4 est **caractérisé par** sa capacité à être en interaction protéine-protéine avec une protéine FBXO28 de pleine longueur.

7. Méthode selon l'une quelconque des revendications 1 à 6, dans laquelle le dérivé ou fragment de FBXO28 est **caractérisé par** sa capacité à être en interaction protéine-protéine avec une protéine PFKFB4 de pleine longueur.

8. Méthode selon la revendication 6 ou 7, dans laquelle la capacité à être en interaction protéine-protéine est la capacité d'une interaction qui imite l'interaction protéine-protéine native entre PFKFB4 et FBXO28.

9. Méthode selon l'une quelconque des revendications 1 à 8, dans laquelle le cancer est un cancer exprimant PFKFB4, de préférence un cancer associé à une expression élevée de PFKFB4, tel que le glioblastome, le cancer du sein, de la prostate ou du poumon.

10. Composé pour utilisation dans une méthode de traitement d'un cancer chez un sujet, la méthode comprenant l'étape d'interrompre dans une cellule associée au cancer chez le sujet l'interaction protéine-protéine entre PFKFB4 et FBXO28.

11. Composé pour utilisation selon la revendication 10, dans lequel le cancer est un cancer **caractérisé par** l'expression de PFKFB4 et FBXO28.

12. Composé pour utilisation selon la revendication 10 ou 11, dans lequel le sujet est un mammifère, de préférence un patient humain souffrant de cancer et nécessitant un traitement.

13. Composé pour utilisation selon l'une quelconque des revendications 10 à 12, dans lequel la méthode comprend l'administration au sujet d'une quantité thérapeutiquement efficace du composé qui réduit spécifiquement l'interaction protéine-protéine entre PFKFB4 et FBXO28 dans une cellule associée au cancer.

14. Composé pour utilisation selon l'une quelconque des revendications 10 à 13, dans lequel le composé est un composé tel qu'identifié selon une méthode de l'une quelconque des revendications 1 à 9.


### SEQUENCE LISTING

<110> Deutsches Krebsforschungszentrum Stiftung des oeffentlichen Rechts
<120> SCREENING METHOD FOR THE IDENTIFICATION OF CANCER THERAPEUTICS
<130> D31602WO
<150> 18153933.9
   <151> 2018-01-29
<160> 2
<170> PatentIn version 3.5
<210> 1
   <211> 469
   <212> PRT
   <213> Homo sapiens
<400> 1
   Met Ala Ser Pro Arg Glu Leu Thr Gln Asn Pro Leu Lys Lys Ile Trp
   1               5                   10                  15
   Met Pro Tyr Ser Asn Gly Arg Pro Ala Leu His Ala Cys Gln Arg Gly
               20                  25                  30
   Val Cys Met Thr Asn Cys Pro Thr Leu Ile Val Met Val Gly Leu Pro
           35                  40                  45
   Ala Arg Gly Lys Thr Tyr Ile Ser Lys Lys Leu Thr Arg Tyr Leu Asn
       50                  55                  60
   Trp Ile Gly Val Pro Thr Arg Glu Phe Asn Val Gly Gln Tyr Arg Arg
   65                  70                  75                  80
   Asp Val Val Lys Thr Tyr Lys Ser Phe Glu Phe Phe Leu Pro Asp Asn
                   85                  90                  95
   Glu Glu Gly Leu Lys Ile Arg Lys Gln Cys Ala Leu Ala Ala Leu Arg
               100                 105                 110
   Asp Val Arg Arg Phe Leu Ser Glu Glu Gly Gly His Val Ala Val Phe
           115                 120                 125
   Asp Ala Thr Asn Thr Thr Arg Glu Arg Arg Ala Thr Ile Phe Asn Phe
       130                 135                 140
   Gly Glu Gln Asn Gly Tyr Lys Thr Phe Phe Val Glu Ser Ile Cys Val
   145                 150                 155                 160
   Asp Pro Glu Val Ile Ala Ala Asn Ile Val Gln Val Lys Leu Gly Ser
                   165                 170                 175
   Pro Asp Tyr Val Asn Arg Asp Ser Asp Glu Ala Thr Glu Asp Phe Met
               180                 185                 190
   Arg Arg Ile Glu Cys Tyr Glu Asn Ser Tyr Glu Ser Leu Asp Glu Asp
           195                 200                 205
   Leu Asp Arg Asp Leu Ser Tyr Ile Lys Ile Met Asp Val Gly Gln Ser
       210                 215                 220
   Tyr Val Val Asn Arg Val Ala Asp His Ile Gln Ser Arg Ile Val Tyr
   225                 230                 235                 240
   Tyr Leu Met Asn Ile His Val Thr Pro Arg Ser Ile Tyr Leu Cys Arg
                   245                 250                 255
   His Gly Glu Ser Glu Leu Asn Leu Lys Gly Arg Ile Gly Gly Asp Pro
               260                 265                 270
   Gly Leu Ser Pro Arg Gly Arg Glu Phe Ala Lys Ser Leu Ala Gln Phe
           275                 280                 285
   Ile Ser Asp Gln Asn Ile Lys Asp Leu Lys Val Trp Thr Ser Gln Met
       290                 295                 300
   Lys Arg Thr Ile Gln Thr Ala Glu Ala Leu Gly Val Pro Tyr Glu Gln
   305                 310                 315                 320
   Trp Lys Val Leu Asn Glu Ile Asp Ala Gly Val Cys Glu Glu Met Thr
                   325                 330                 335
   Tyr Glu Glu Ile Gln Asp Asn Tyr Pro Leu Glu Phe Ala Leu Arg Asp
               340                 345                 350
   Gln Asp Lys Tyr Arg Tyr Arg Tyr Pro Lys Gly Glu Ser Tyr Glu Asp
           355                 360                 365
   Leu Val Gln Arg Leu Glu Pro Val Ile Met Glu Leu Glu Arg Gln Glu
       370                 375                 380
   Asn Val Leu Val Ile Cys His Gln Ala Val Met Arg Cys Leu Leu Ala
   385                 390                 395                 400
   Tyr Phe Leu Asp Lys Ala Ala Glu Gln Leu Pro Tyr Leu Lys Cys Pro
                   405                 410                 415
   Leu His Thr Val Leu Lys Leu Thr Pro Val Ala Tyr Gly Cys Lys Val
   420 425 430
   Glu Ser Ile Phe Leu Asn Val Ala Ala Val Asn Thr His Arg Asp Arg
   435 440 445
   Pro Gln Asn Val Asp Ile Ser Arg Pro Pro Glu Glu Ala Leu Val Thr
   450 455 460
   Val Pro Ala His Gln
   465
<210> 2
   <211> 368
   <212> PRT
   <213> Homo sapiens
<400> 2
   Met Ala Ala Ala Ala Glu Glu Arg Met Ala Glu Glu Gly Gly Gly Gly
   1 5 10 15
   Gln Gly Asp Gly Gly Ser Ser Leu Ala Ser Gly Ser Thr Gln Arg Gln
   20 25 30
   Pro Pro Pro Pro Ala Pro Gln His Pro Gln Pro Gly Ser Gln Ala Leu
   35 40 45
   Pro Ala Pro Ala Leu Ala Pro Asp Gln Leu Pro Gln Asn Asn Thr Leu
   50 55 60
   Val Ala Leu Pro Ile Val Ala Ile Glu Asn Ile Leu Ser Phe Met Ser
   65 70 75 80
   Tyr Asp Glu Ile Ser Gln Leu Arg Leu Val Cys Lys Arg Met Asp Leu
   85 90 95
   Val Cys Gln Arg Met Leu Asn Gln Gly Phe Leu Lys Val Glu Arg Tyr
   100 105 110
   His Asn Leu Cys Gln Lys Gln Val Lys Ala Gln Leu Pro Arg Arg Glu
   115 120 125
   Ser Glu Arg Arg Asn His Ser Leu Ala Arg His Ala Asp Ile Leu Ala
   130 135 140
   Ala Val Glu Thr Arg Leu Ser Leu Leu Asn Met Thr Phe Met Lys Tyr
   145 150 155 160
   Val Asp Ser Asn Leu Cys Cys Phe Ile Pro Gly Lys Val Ile Asp Glu
                   165                 170                 175
   Ile Tyr Arg Val Leu Arg Tyr Val Asn Ser Thr Arg Ala Pro Gln Arg
               180                 185                 190
   Ala His Glu Val Leu Gln Glu Leu Arg Asp Ile Ser Ser Met Ala Met
           195                 200                 205
   Glu Tyr Phe Asp Glu Lys Ile Val Pro Ile Leu Lys Arg Lys Leu Pro
       210                 215                 220
   Gly Ser Asp Val Ser Gly Arg Leu Met Gly Ser Pro Pro Val Pro Gly
   225                 230                 235                 240
   Pro Ser Ala Ala Leu Thr Thr Met Gln Leu Phe Ser Lys Gln Asn Pro
                   245                 250                 255
   Ser Arg Gln Glu Val Thr Lys Leu Gln Gln Gln Val Lys Thr Asn Gly
               260                 265                 270
   Ala Gly Val Thr Val Leu Arg Arg Glu Ile Ser Glu Leu Arg Thr Lys
           275                 280                 285
   Val Gln Glu Gln Gln Lys Gln Leu Gln Asp Gln Asp Gln Lys Leu Leu
       290                 295                 300
   Glu Gln Thr Gln Ile Ile Gly Glu Gln Asn Ala Arg Leu Ala Glu Leu
   305                 310                 315                 320
   Glu Arg Lys Leu Arg Glu Val Met Glu Ser Ala Val Gly Asn Ser Ser
                   325                 330                 335
   Gly Ser Gly Gln Asn Glu Glu Ser Pro Arg Lys Arg Lys Lys Ala Thr
               340                 345                 350
   Glu Ala Ile Asp Ser Leu Arg Lys Ser Lys Arg Leu Arg Asn Arg Lys
           355                 360                 365